# EUROPEAN PATENT SPECIFICATION

(11) **EP 4 647 477 B1**
(45) Date of publication and mention of the grant of the patent: **23.09.2026**
(21) Application number: 24174739.3
(22) Date of filing: 08.05.2024
(51) Int. Cl.: C10G 31/10, B01D 17/02, C07C 29/86, C07C 31/04

(54) **METHOD FOR CLEANING FUELS ONBOARD A SHIP**
VERFAHREN ZUR REINIGUNG VON BRENNSTOFFEN AN BORD EINES SCHIFFES
PROCÉDÉ DE NETTOYAGE DE CARBURANTS À BORD D'UN NAVIRE

(43) Date of publication of application: 12.11.2025
(73) Proprietor: Alfa Laval Corporate AB, 221 00 Lund (SE)
(72) Inventor: DE WITTE, Henricus, 148 96 Sorunda (SE)
(74) Representative: Alfa Laval Attorneys

(56) References cited:
- CN-A- 112 555 073
- CN-B- 103 409 177
- US-A1- 2019 022 555
- US-A1- 2021 275 943

## Description

### Field of the Invention

The present invention relates to disc-stack centrifugal separators, and more specifically to a method for cleaning fuels onboard a ship.

### Background of the Invention

US 2021/275943 A1 discloses a method for cleaning a fuel oil for a diesel engine on board a ship. It uses a centrifugal separator, such as a disc-stack centrifuge. US 2019/022555 A1 discloses a method and system for cleaning oily waste comprising water, solids and a fuel oil on board of a ship. The system uses disc-stack centrifugal separation. CN 103 409 177 B discloses a marine fuel oil comprising methanol. CN 112 555 073 A discloses a diesel-methanol dual-fuel system for marine diesel engines including a switch to operate the engine in a diesel-methanol dual-fuel mode or the original diesel mode.

Heavy fuel oil (HFO) and diesel oil (DO) have been the most common fuel oils used for commercial vessels. Fuels used onboard ships is usually treated on board before use in the engine in order to remove solid contaminants, such as particles of silicon and aluminium compounds (e.g. microporous aluminium silicates or aluminosilicates known as zeolites), called catalyst fines, and liquid contaminants such as water. Marine diesel engines usually accept several types of commercially available fuel oils as long as they are adequately treated on board the ship. However, the use of HFO and diesel fuels has a negative environmental impact, and the maritime industry is exploring alternative fuels and technologies to reduce emissions as part of the overall decarbonization of shipping.

Currently biofuels, such as fatty acid methyl esters (FAMEs) and hydrogenated vegetable oil (HVO), can be utilized as drop-in alternatives to these conventional fuels, either as complete substitutes or as blends with the common fossil-based marine fuels. Methanol (MeOH) is also a strong candidate for an alternative fuel as it can be produced in a sustainable electrolyser process or from bio resources.

Ships engines may be designed as 'Dual Fuel' engines, allowing them to run on both MeOH and DO/FAME/HVO, from a usage point of view called hydrocarbon fuels. However, introducing a mix of these fuels to the engine at the same time may potentially create serious engine damage, which is why the engine is operated in two different operational modes; one in which MeOH is used as fuel and one in which DO/FAME/HVO is used as fuel. This also means that the fuel supply systems going from the fuel tanks to the engine are designed as two separate systems.

Further, there are also dedicated fuel tanks for MeOH and DO, but since sustainable MeOH is not always available, MeOH takes twice the amount of space per energy unit as DO/FAME/HVO and tank space is limited onboard a ship, having dedicated MeOH or DO tanks may waste a lot of valuable space onboard. Thus, there is a need in the art for improved methods and systems for using MeOH as a fuel onboard a ship.

### Summary of the Invention

A main object of the present invention is to provide a system and method for using fuel oils, including methanol, onboard a ship. Further, there is an object of the present invention to provide a method for cleaning fuel that allows for flexible use of tank space onboard a ship.

As a first aspect of the invention, there is provided a method according to claim 1.

The first aspect of the invention is based on the insight that the need onboard ship of utilizing a fuel tank for both methanol and another type of fuel may be realized using a disc-stack centrifugal separator. When a fuel tank is emptied to the maximum extent possible before being filled with another liquid, there will always be some liquid remaining since it is practically impossible to fully empty a tank. However, as the inventor has realised, a disc-stack centrifugal separator may solve the task of both separating a fraction of another fuel from methanol (step a) and separating a fraction of methanol from that other fuel (step b). As experiments have shown, the use of a disc-stack separator is able to perform the separation task to provide a good-enough separation, even though the fuels may be solvable to some degree one in another.

Consequently, with the method of the first aspect of the invention, the same tank may be used for methanol and another type of fuel onboard a ship, thereby saving space and thus providing for the practical use of having methanol as an alternative fuel onboard a ship.

A centrifugal separator is generally used for separation of liquids and/or solids from a liquid mixture or a gas mixture. During operation, a fluid mixture to be separated is introduced into a rotating bowl and due to the centrifugal forces, the "heavy phase" of higher density accumulates at the periphery of the rotating bowl whereas the "light phase" of lower density accumulates closer to the central axis of rotation. This allows for collection of the separated fractions, e.g. by means of outlets arranged at different radii from the rotational axis. In the present disclosure, the "liquid light phase" refers to the liquid phase of low density, whereas the "liquid heavy phase" refers to the liquid phase of high density.

A "disc-stack centrifugal separator" is a centrifugal separator in which there are surface enlarging inserts within the separator bowl to increase the separating area. These inserts may be a stack of separation discs, such as a stack of frustoconical separation discs. The number of discs in the disc-stack may be at least 50, such as at least 100, such as at least 150.

The disc stack separator may be a solid wall bowl centrifugal separator, in which separated solids accumulate within the centrifuge bowl, or a centrifugal separator arranged for intermittently discharging a separated solid phase from the centrifuge bowl.

Step a) of separating a fraction of first fuel residues from a methanol fuel may also comprise the step of supplying the methanol fuel to the disc-stack centrifugal separator and discharging a cleaned methanol phase and a first fuel residues as a separate phase from the disc-stack centrifugal separator. During the separation in step a), the first fuel residues are present in much less quantity than the methanol fuel.

In analogy, step b) of separating a fraction of methanol fuel residues from a first fuel may also comprise the step of supplying the first fuel to the disc-stack centrifugal separator and discharging a cleaned first fuel phase and a methanol fuel residues as a separate phase from the disc-stack centrifugal separator. During the separation in step b), the methanol fuel residues are present in much less quantity than the first fuel.

The "first fuel" is a fuel suitable for an engine onboard a ship. The first fuel may be a hydrocarbon fuel. As an example, the first fuel may be a diesel fuel, a biofuel, such as a biodiesel, or a Heavy Fuel Oil (HFO), or any blend thereof.

The first fuel may be as defined in ISO 8217, Petroleum products - Fuels (class F) - Specification of marine fuels, edition 2017, or an oil component/phase originating from the pre-treatment of such an oil before use in an engine on board a ship. The "first fuel" may thus be marine (residual) fuel oil (MFO), according to Table 2 of this standard, or diesel oil according to Table 1 of this standard, or biofuels as per paragraph 5.1. The "first fuel" may be composed of different types of fuel oils having different viscosities, generally stored in a tank, which means that the type of first fuel in steps a) and b) may differ between separation or during a separation process.

Thus, in embodiments of the first aspect, the first fuel is a diesel oil or a biofuel, or any blend thereof.

The biofuel may be selected from Fatty Acid Methyl Esters (FAME) blends and Hydrogenated Vegetable Oil (HVO)

Furthermore, the "first fuel" may have a density that is higher than the methanol fuel. This is usually the case for a majority of "first fuels," such as for diesel oil and most biofuels. In such case, the centrifugal separator is operated as a purifier in step a) and as a concentrator in step b).

However, the "first fuel" may have a density that is lower than the methanol fuel. This may be the case e.g. if Hydrogenated Vegetable Oil (HVO) is used as first fuel. In such case, the centrifugal separator is operated as a concentrator in step a) and as a purifier in step b)

Consequently, the method of the first aspect comprises switching operation mode from purifier to concentrator, or vice versa, between steps a) and b). This may be performed in several ways, as further discussed below.

A "purifier" is a centrifugal separator for liquid - liquid - solid separation, which separates two liquids of different densities, and any solids present, from each other. In a purifier, the light liquid phase - i.e. the liquid phase of lowest density- is typically the large fraction which is meant to cleaned.

A "concentrator" is a centrifugal separator for liquid - liquid - solid separation, which separates two liquids of different densities, and any solids present, from each other. In a concentrator, the liquid heavy phase - i.e. the liquid phase of highest density- is typically the large fraction which is meant to cleaned.

Thus, a "purifier" separator, or a separator operated as a purifier, may be used in step a) when methanol is the large fraction and the first fuel has a density that is higher than the methanol fuel, or in step b) when the first fuel is the large fraction, and the first fuel has a density that is lower than the methanol fuel.

In analogy, a "concentrator" separator, or a separator operated as a concentrator, may be used in step b) when methanol is the small fraction and the first fuel has a density that is higher than the methanol fuel, or in step a) when methanol is the large fraction, and the first fuel has a density that is lower than the methanol fuel.

Both the purifier and concentrator may be in the form of a solid wall bowl centrifugal separator or a separator for intermittent discharge of a solid phase.

In embodiments of the first aspect, the separation in step a) and step b) is performed with the same disc-stack centrifugal separator. Consequently, step a) may comprise separating a fraction of first fuel residues from a methanol fuel with a first disc-stack centrifugal separator, thereby providing a cleaned methanol fuel onboard a ship; and step b) may comprise separating a fraction of methanol fuel residues from a first fuel with the same first disc-stack centrifugal separator, thereby providing a cleaned first fuel onboard said ship.

Using a single separator to perform the separation tasks in steps a) and b) may thus save space onboard the ship.

As an example, when the same centrifugal separator is used for steps a) and b), the method may comprise changing the disc-stack in the disc-stack centrifugal separator for switching operation of the disc-stack centrifugal separator as purifier to operation of the disc-stack centrifugal separator as a concentrator, and vice versa, between steps a) and b).

The disc-stacks of a purifier and a concentrator may differ. A separation disc in the stack usually has a through hole or cut out such that axially extending distribution channels are formed in the stack of discs. Such distribution channels are for distributing the liquid feed mixture within the stack. In a purifier, in which there is maximum cleaning of the liquid light phase, such axial distribution channels in the "purifier disk-stack" are arranged closer to the periphery of the disc-stack, such as within the outer half of the radial extension of the disc-stack. In a concentrator, in which there is maximum cleaning of the heavy phase, such axial distribution channels in the "concentrator disk-stack" are arranged closer to the axis of rotation, such as within the inner half of the radial extension of the disc-stack.

Thus, "operating the disc-stack centrifugal separator as a purifier" may comprise operating the disk-stack centrifugal separator with a purifier disc-stack. In analogy, "operating the disc-stack centrifugal separator as a concentrator" may comprise operating the disk-stack centrifugal separator with a concentrator disc-stack.

The method may thus comprise a step of substituting the purifier disk-stack to a concentrator disk-stack, or vice versa, between steps a) and b).

However, switching from operating the separator as a purifier to operating the separator as a concentrator may be performed without changing the disc-stack. As an example, the method may comprise changing operational parameters for switching operation of the disc-stack centrifugal separator as a purifier to operation of the disc-stack centrifugal separator as a concentrator, and vice versa. Such operational parameters may be the flow of the liquid mixture that is separated, the backpressure on the outlet for the separated liquid light phase and/or the back pressure on the outlet for the separated liquid heavy phase.

Furthermore, steps a) and b) do not necessarily need to be performed using the same disc-stack centrifugal separator. Consequently, in embodiments of the first aspect, separation in step a) and step b) are performed with different disc-stack centrifugal separators. Thus, the disc-stack centrifugal separator may actually be a purifier when the centrifugal separator is operated as a purifier and the disc-stack centrifugal separator may be a concentrator when the centrifugal separator is operated as a concentrator.

In embodiments of the first aspect, at least one of steps a) or b) is performed at a temperature of 11 °C or lower. As an example, only step a), or only step b), or both of steps a) and b) may be performed at a temperature of 11 °C or lower, such as at a temperature of 10 °C or lower, such as at a temperature of 8 °C or lower. This may reduce the risk of a fire during separation since the flash point of methanol is around 11 °C. Thus, separating the methanol fuel at a low temperature may be for the purpose of creating an explosion safe process. Separation at such low temperature may be performed by e.g. lowering the temperature of the liquid that is supplied to the separator, i.e. the liquid feed mixture that is to be separated, or by cooling down the separator, or parts of the separator, as such.

In embodiments of the first aspect, the methanol fuel that is cleaned in step a) is supplied from a storage tank in which previously said first fuel has been stored, and wherein the first fuel that is cleaned in step b) is supplied from a storage tank in which previously said methanol fuel has been stored.

The storage tank in which previously the first fuel has been stored and the storage tank in which previously the methanol fuel has been stored does not necessarily have to be the same storage tank onboard a ship, since the ship may comprise a plurality of storage tanks for different fuels.

However, in embodiments of the first aspect, the methanol fuel that is cleaned in step a) and the first fuel that is cleaned in step b) is supplied to the disc-stack centrifugal separator from the same storage tank.

As discussed above, using the same storage tank for both the methanol fuel and the first fuel may save valuable space onboard the ship.

A fuel to be cleaned may be withdrawn from different portions of the storage tank. As an example, when the first fuel has a density that is higher than the density of the methanol fuel, the fuel to be cleaned may initially be withdrawn from a bottom portion of the storage tank in step a) and initially skimmed from a top portion of the storage tank in step b).

When the first fuel has a higher density than the methanol fuel, such fuel will accommodate at the bottom layer of the tank. During step a), the first fuel residue is therefore most easily removed by using a 'low suction' of the storage tank. Further, during step b), any methanol residue will accumulate on the top layer in the storage tank and may therefore most easily be removed by 'skimming' the top layer of the tank, using for example a skimming buoy or a tank outlet at top height. With such a setup, the most contaminated portion of the fuel to be cleaned is initially sent to the centrifugal separator for cleaning, and therefore, most of the residue is cleaned in separation favourable circumstances before doing deep cleaning of the fuel.

Skimming is withdrawing fuel from the upper contents of the storage tank. An "upper portion" of the contents present in the storage tank may be the portion representing the upper 50%, such as the upper 30 %, such as the upper 20 % of the contents present in the storage tank. In analogy, a "lower portion" of the contents present in the storage tank may be the portion representing the lower 50%, such as the lower 30 %, such as the lower 20 % of the contents of the storage tank. "Upper" and "lower" thus refers to positions along the vertical extension of the storage tank.

In analogy with the above example, when the first fuel has a density that is lower than the density of the methanol fuel, the fuel to be cleaned may initially be skimmed from a top portion of the storage tank in step a) and initially withdrawn from a bottom portion of the storage tank in step b).

As a further example, the method may comprise emptying the storage tank from methanol fuel and refilling said storage tank with the first fuel between steps a) and b).

The methanol fuel may be emptied from the bottom of the storage tank. However, since complete emptying is practically nearly impossible, a fraction of methanol may be present in the storage tank when refilling the tank. Such fraction of methanol may then be cleaned from the first fuel during step b).

In embodiments of the first aspect, the method is further comprising repeating steps a) and b) onboard the ship.

Consequently, the method may comprise switching between the two separation processes several times. Thus, if the same tank is used for both fuels, the method may comprise emptying the storage tank from the first fuel and refilling said storage tank with the methanol fuel after step b). The fraction of the first fuel that is still present in the storage tank may consequently be separated from the methanol fuel during a subsequent separation when repeating step a).

In embodiments of the first aspect, step a) is further comprising collecting the cleaned methanol fuel in a methanol service tank. The separated first fuel fraction may be collected in a first fuel slop tank. In analogy, step b) may comprise collecting the cleaned first fuel in a first fuel service tank. The separated methanol fraction may be collected in a methanol slop tank.

From the service tanks, the cleaned fuels may be used as fuels for the engine by fuel supply systems installed onboard the ship.

As a second aspect of the invention, there is provided a system for cleaning fuels onboard a ship, wherein the system is suitable for carrying out the method according to the first aspect discussed above. Such system is comprising a disc-stack centrifugal separator for separating a fraction of first fuel residues from a methanol fuel with a disc-stack centrifugal separator, thereby providing a cleaned methanol fuel onboard a ship. The same centrifugal separator may further be arranged for separating a fraction of methanol fuel residues from a first fuel with a disc-stack centrifugal separator, thereby providing a cleaned first fuel onboard said ship. The system further comprises a storage tank for fuel to be cleaned. As an example, the system may be configured for supplying contents from both the upper and the lower portions of the contents present in the storage tank to the centrifugal separator.

The terms and definitions used in relation to the second aspect are the same as discussed in relation to the first aspect above.

The centrifugal separator may thus be as discussed in relation to the first aspect above. The centrifugal separator may thus be configured to be operated both as a purifier and a concentrator. The centrifugal separator may be arranged for separating two liquid phases and possibly also a solid phase from the liquid feed mixture. The two liquid phases may be discharged continuously. The centrifugal separator may comprise a stationary frame and a drive member configured to rotate a centrifuge bowl within the frame. The centrifuge bowl is enclosing the separation space. The separation space comprises a stack of separation discs arranged centrally around the axis of rotation. Such separation discs form surface enlarging inserts in the separation space. The separation discs may have the form of a truncated cone, i.e. the stack may be a stack of frustoconical separation discs.

### Brief description of the Drawings

The above, as well as additional objects, features, and advantages of the present inventive concept, will be better understood through the following illustrative and nonlimiting detailed description, with reference to the appended drawings. In the drawings like reference numerals will be used for like elements unless stated otherwise.
Figure 1 shows a schematic drawing of an embodiment of a system for cleaning fuels onboard a ship in which the method of the present invention may be performed.
Figure 2 shows a schematic drawing of an embodiment of a system for cleaning fuels onboard a ship in which the method of the present invention may be performed.
Figure 3 schematically illustrates a method of cleaning fuels onboard a ship according to the present invention.
Figure 4 shows a schematic drawing of an embodiment of a centrifugal separator of the system for cleaning fuels onboard a ship.

### Detailed Description

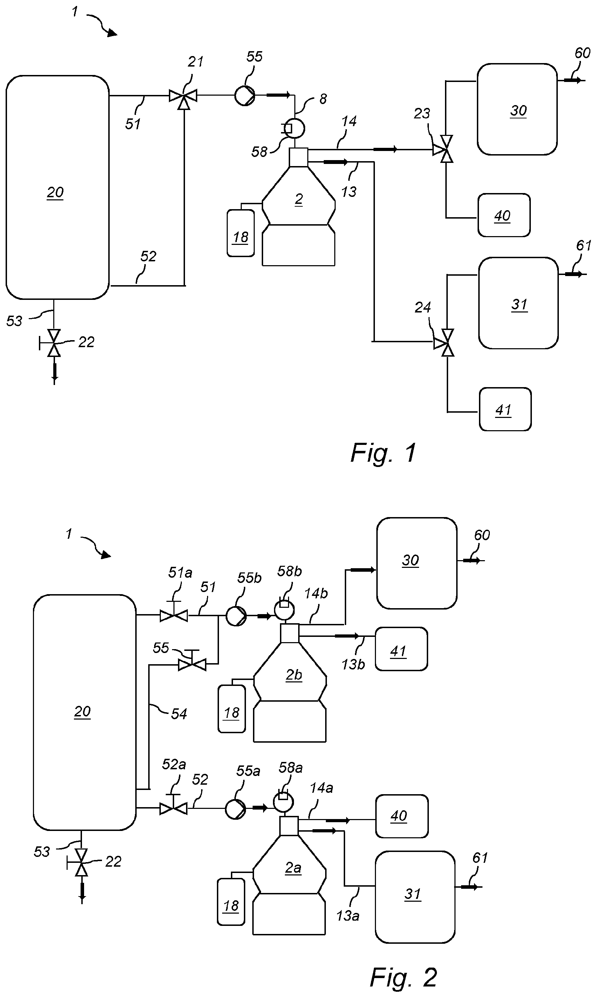

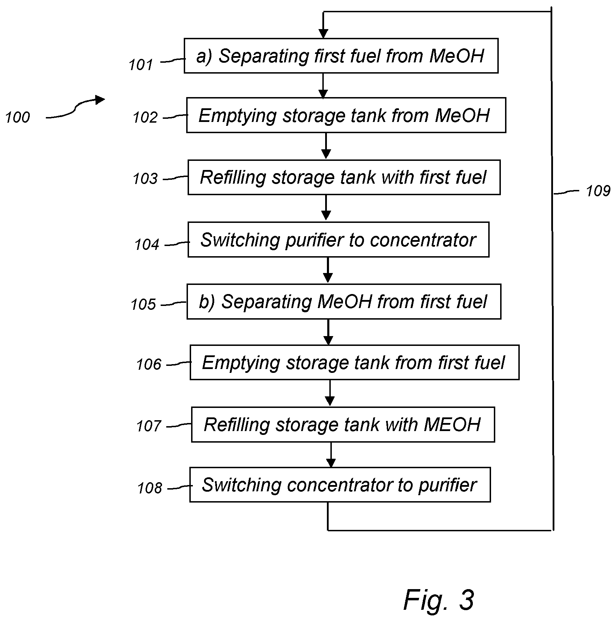

The method and the system according to the present disclosure will be further illustrated by the following description with reference to the accompanying drawings. In the examples of Figs. 1 and 2, and in the method illustrated in Fig. 3, the first fuel is a hydrocarbon fuel, such as a diesel oil or a biofuel, having density that is higher than the density of methanol fuel. For most hydrocarbon fuels this is the case, with an exception for Hydrogenated Vegetable Oil (HVO) fuels that may have a density that is lower than the density of the methanol fuel.

Fig. 1 shows a schematic drawing of an embodiment of a system 1 for cleaning fuels onboard a ship. The system comprises a disc-stack centrifugal separator 2, which is further discussed in relation to Fig. 3 below. Fuel to be cleaned is supplied from storage tank 20. In this example, the system 1 comprises a single storage tank for fuel, but it is to be understood that the system 1 may comprise a plurality of storage tanks 20 for different fuels.

During a first mode of operation (step a)), methanol fuel is to be cleaned from residues of the hydrocarbon fuel. Fuel from storage tank 20 is then supplied to the separator 2 from a lower portion of the content of the storage tank, i.e. the lower layer in the storage tank 20 is withdrawn via line 52. Inlet pump 55 is used to supply the fuel to the separator 2 via the inlet line 8. Further, the fuel to be cleaned passes cooler 58, which is arranged to cool the fuel to be cleaned down to a temperature of 11 °C or lower to reduce the risk of an explosion during separation, thus providing an alternative for explosion safe measures such as nitrogen blanketing to be taken. In the disc-stack centrifugal separator 2 a fraction of hydrocarbon fuel residues is separated from the methanol. The hydrocarbon fuel residues originate from a hydrocarbon fuel that has a density that is higher than the density of methanol fuel and which was previously stored in tank 20. Due to the higher density of this hydrocarbon fuel, a major portion of any residues of the hydrocarbon fuel will in time form a phase in the lower portion of the tank 20, which is why a high amount of such residues will be present during the initial separation in the separator 2 when withdrawing fuel to be cleaned from a lower portion of the storage tank.. The centrifugal separator 2 is in this mode (step a)) operated as a purifier.

The cleaned methanol fuel is discharged from the centrifugal separator 2 via a liquid light phase outlet of the separator to light phase outlet line 13. Valve 24 directs the cleaned methanol fuel to the service tank 31 for methanol. From this tank 31, methanol may be withdrawn when needed to the engine of the ship, such as to a fuel supply system (not shown), via outlet 61. Separated residues of the hydrocarbon fuel is in this mode of operation (step a)) discharged from the centrifugal separator 2 via a liquid heavy phase outlet of the separator to heavy phase outlet line 14. Valve 23 directs the separated residues of the hydrocarbon fuel to slop tank 40. Any separated solid phase may be intermittently discharged from the centrifuge bowl and collected in sludge storage tank 18, which is in the form of a cyclone.

When a desired amount of methanol fuel in tank 20 has been cleaned, the tank 20 is emptied using valve 22 and the lower emptying line 53. The contents of the tank may be emptied to e.g. one of the slop tanks 40, 41 or another slop tank in the system. After such an emptying of the storage tank 20, it is refilled with a hydrogen fuel of higher density than the methanol.

Thereafter, during a second mode of operation (step b)), the hydrocarbon fuel is supplied to the centrifugal separator 2. Before the separation process in this second mode of operation (step b)), the separator 2 is changed to operate as a concentrator. This may be performed by e.g. changing the disc-stack in the centrifugal separator 2 from a purifier disc-stack to a concentrator disc-stack. At least initially, fuel to be cleaned is in this mode supplied from the top layer of the contents of the storage tank. This is performed by 'skimming' the top layer of the storage tank 20, using for example a skimming buoy or a tank outlet at top height of the storage tank. Any methanol residues still present in the storage tank will mostly be present on top of the hydrocarbon fuel (due to the lower density), which is why initially the fuel to be cleaned is skimmed from the upper portion of the contents of the storage tank 20 via supply line 51.The skimmed portion is supplied to the separator 2 by shifting valve 21 and using supply pump 55. However, after a certain period of time, when it is believed that the residue layer of methanol fuel has been removed by the skimming, fuel to be cleaned may be supplied during the second mode of operation by withdrawing fuel from the lower portion of the tank and shifting three-way valve 21.

Also during this second mode (step b)), the fuel to be cleaned is cooled down to a temperature of 11 °C or lower to reduce the risk of an explosion during separation.

During the second mode of operation (step b)), the cleaned hydrocarbon fuel is discharged via a liquid heavy phase outlet of the centrifugal separator 2 to heavy phase outlet line 14. Valve 23 is used to direct the cleaned hydrocarbon fuel to service tank 30 used for the hydrocarbon fuel. From this tank 30, the hydrocarbon fuel may be withdrawn when needed to the engine of the ship, such as to a fuel supply system (not shown), via outlet 60. Separated residues of methanol is in this mode of operation (step b)) discharged from the centrifugal separator 2 via a liquid light phase outlet of the separator to light phase outlet line 13. Valve 24 directs the separated residues of methanol to slop tank 41.

Thereafter, the storage tank 20 may be emptied from the hydrocarbon fuel - using valve 22 and emptying line 53 - and then be refilled with methanol fuel before a subsequent first mode of operation (step a)) is performed.

The method of the present invention may however be performed in a system comprising two centrifugal separators. Such an embodiment is shown in Fig. 2. This system 1 for cleaning fuels onboard a ship function in the same way as the system 1 discussed in relation to Fig. 1, but with a few differences discussed below.

During the first mode of operation (step a)), methanol fuel is supplied from the lower portion of the content of the storage tank 20 in supply line 52 to a purifier centrifugal separator 2a using a first supply pump 55a. The methanol fuel is cooled before separation using cooler 58a. During this step, cleaned methanol fuel is discharged to methanol service tank 31 and the separated fraction of residues of the hydrocarbon fuel is discharged to the slop tank 40.

Then, after the storage tank 20 has been emptied as much as possible (using valve 22 and emptying line 53) and refilled with the hydrocarbon fuel, the second mode of operation (step b)) is initiated. The hydrocarbon fuel to be cleaned from methanol residues is then initially supplied from the upper portion of the contents of the storage tank 20 in line 51 to a concentrator centrifugal separator 2b using a second supply pump 55b. The hydrocarbon fuel is cooled before separation using cooler 58b. After a certain period of time, when it is believed that the upper layer of the contents of the tank has been skimmed, fuel may be supplied to the concentrator centrifugal separator 2b from the lower portion of the tank 20, as indicated by line 54 and valve 55 in Fig. 2,

Valve 51a in the fuel line 51 and valve 52a in fuel line 52 may be used to open and close the fuel lines when needed. Such valves may also be present in fuel lines 51 and 52 in the system discussed in relation to Fig. 1 above. Thus, during the first mode of operation (step a)), valve 52a is open and valve 51a is closed and during the second mode of operation (step b)), valve 52a is closed and valve 51a is initially open during the skimming process. If fuel is to be supplied form the lower portion of tank 20 during the second mode of operation, valve 51a may be closed and valve 55 open. From the concentrator centrifugal separator 2b, cleaned hydrocarbon fuel is discharged to the service tank 30 for the hydrocarbon fuel, whereas the fraction of methanol residues is discharged to the slop tank 41 for methanol.

The first separation process as discussed in relation to Figs. 1 and 2, i.e. the separation of a fraction of first fuel residues from a methanol fuel, and possibly also the second separation process, i.e. the separation a fraction of methanol fuel residues from a first fuel, may be performed at low temperature, such as at a temperature of 11 °C or lower. As an example, any of the separation processes may be performed at a temperature of 10 °C or lower, such as at a temperature of 8 °C or lower. This may decrease the risk of the methanol fuel or methanol fuel residues catching fire. Thus, also the system as discussed in relation to Fig. 2 may comprise a cooler for cooling the fuel to be cleaned, such as a cooler upstream of purifier separator 2a and/or a cooler upstream concentrator separator 2b.

An embodiment of the method 100 of the present invention for cleaning fuels onboard a ship is schematically illustrated in Fig. 3. In this embodiment, the first fuel has a density that is higher than the density of the methanol fuel. As also demonstrated in the Figs. 1 and 2, the method may comprise the steps of:
- step a) of separating 101 a fraction of first fuel residues from a methanol fuel with a disc-stack centrifugal separator, thereby providing a cleaned methanol fuel onboard a ship
- step b) of separating 105 a fraction of methanol fuel residues from a first fuel with a disc-stack centrifugal separator, thereby providing a cleaned first fuel onboard said ship.

Between steps a) and b), the following steps may be performed:
- Emptying 102 the storage tank from any methanol (MeOH) that is left in the storage tank. In steps a) and b) above, the methanol fuel and the first fuel may originate from the same storage tank.
- Refilling 103 the storage tank with the first fuel.
- Switching 104 operating the centrifugal separator as a purifier to operating the separator as a concentrator. This may be performed by changing the disc-stack from a purifier disk-stack in step a) to a concentrator disk-stack in step b) or controlling any operational parameters of the separation process. As an alternative, different centrifugal separators may be used, i.e. a purifier in step a) and a concentrator in step b).

After step b) has been performed, the following steps may be performed:
- Emptying 106 the storage tank from any first fuel that is left in the storage tank.
- Refilling 107 the storage tank with the methanol fuel.
- Switching 108 operating the centrifugal separator as a concentrator to operating the separator as a purifier. This may be performed by changing the disc-stack from a concentrator disk-stack to a purifier disk-stack in step b) or controlling any operational parameters of the separation process. As an alternative, different centrifugal separators may be used, i.e. a purifier in step a) and a concentrator in step b).

Then, the method 100 may comprise repeating 109 steps a) and b), or any of the above-mentioned steps, onboard the ship.

In the above examples discussed in relation to Figs. 1-3, the first fuel has a density that is higher than the methanol fuel. However, there may be embodiments in which the first fuel has a density that is lower than the methanol fuel. This may be the case when Hydrogenated Vegetable Oil (HVO) is used. In such case, the disc-stack centrifugal separator 2 is operated as a concentrator during the first mode of operation (in step a)) and as a purifier during the second mode of operation (in step b)).

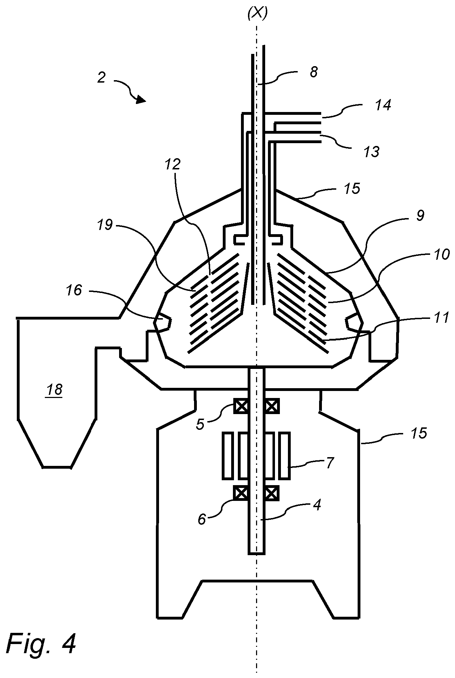

Fig. 4 illustrates and gives some further details of an example of a centrifugal separator 2 that may be used in the system 1 as shown in Figs. 1 and 2. This centrifugal separator 2 has an intermittent discharge system for intermittently discharging any separated solids from the centrifuge bowl. However, the centrifugal separator may as an alternative be a solid wall bowl centrifugal separator, in which any separated solid phase accumulates within the centrifuge bowl.

The centrifugal separator 2 comprises a rotatable centrifuge bowl 9 that forms within itself a separation chamber 10 in which centrifugal separation of the fuel takes place during operation.

The separation chamber 10 is provided with a disc-stack 19 of frustoconical separation discs 11 in order to achieve effective cleaning of the fuel. The stack 19 of truncated conical separation discs 11 are examples of surface-enlarging inserts. These discs 11 are fitted centrally and coaxially with the centrifuge bowl and may comprise holes which form axial distribution channels 12 for axial flow of liquid when the separation discs 11 are fitted as the stack 19 in the centrifugal separator 2. The disc-stack 19 may be a purifier disc-stack when the centrifugal separator is operated as a purifier in the method 100. In a purifier disc-stack, the axial distribution channels 12 are arranged closer to the periphery of the disc-stack 19, such as within the outer half of the radial extension of the disc-stack. The disc-stack 19 may however be a concentrator disc-stack when the centrifugal separator is operated as a concentrator in the method 100. In a concentrator disc-stack, the axial distribution channels 12 are arranged closer to the axis of rotation (X), such as within the inner half of the radial extension of the disc-stack 19.

The inlet pipe 8 forms a central duct and is thus arranged for introducing the fuel to be cleaned to the centrifuge bowl 9. The fuel is in this embodiment supplied from the top but also separators that are fed from the bottom may be used in the method 100 of the present invention.

The centrifugal separator 2 has extending from it a liquid light phase outlet 13 for a lower density component (liquid light phase) separated from the fuel, and a liquid heavy phase outlet 14 for a higher density component (liquid heavy phase) separated from the fuel. The outlets 13 and 14 extend through the frame 15.

The centrifuge bowl 9 is further provided at its outer periphery with a set of radial sludge outlets 16 in the form of intermittently openable outlets for discharge of higher density component such as sludge or other solids in the fuel that is cleaned. This material is thus discharged from a radially outer portion of the separation chamber 10 to the space around the centrifuge bowl 9 and further collected in sludge tank 18, which is in the form of a cyclone.

The centrifugal separator 2 is further provided with a drive motor 7 for rotating the spindle 4 and the centrifuge bowl 9 attached to the spindle at a desired speed. The spindle 4 and the centrifuge bowl 9 is suspended in the stationary frame 15 by upper 5 and lower 6 bearings. As an alternative, drive motor 7 can also be mounted outside the separator 1 and arranged for driving the spindle 4 via a belt.

During operation of the separator in Fig. 4, the centrifuge bowl 9 is caused to rotate by the drive motor 7. Via the inlet pipe 8, fuel to be cleaned is brought into the separation space 10. Depending on the density, different phases in the fuel is separated between the separation discs 11. The heavy phase moves radially outwards between the separation discs, whereas the light phase moves radially inwards between the separation discs and is forced through outlet 13 arranged at a radial inner level in the centrifuge bowl 9. The liquid of higher density is instead forced out through outlet 14 that is arranged at position that is at a radial distance that is larger than the radial position of outlet 13. Thus, during separation, an interphase between the liquid of lower density and the liquid of higher density is formed in the separation space 10. Solids, or sludge, accumulate at the periphery of the separation chamber 10 and is emptied intermittently from the separation space by the sludge outlets 16 being opened, whereupon sludge and a certain amount of fluid is discharged from the separation space by means of centrifugal force.

### Experimental example

To demonstrate that methanol fuel (MeOH) and hydrocarbon fuels are actually separable even though MeOH may be dissolvable in the hydrocarbon fuel and vice versa, a spin test was set up.

Samples investigated were:
- (98%/2%) mixtures of MeOH with diesel oil (DO) or a biodiesel (Hydrogenated Vegetable Oil (HVO) with or without addition of Fatty Acid Methyl Esters (FAME) blends)
- (98%/2%) mixtures of diesel oil (DO) or the above biodiesel with MeOH
- (90%/10%) mixtures of MeOH with diesel oil (DO) or a biodiesel (Hydrogenated Vegetable Oil (HVO) with or without addition of Fatty Acid Methyl Esters (FAME) blends)
- (90%/10%) mixtures of diesel oil (DO) or the above biodiesel with MeOH

The samples were prepared by mixing to simulate mixing in a tank and the mixtures were observed immediately after the mixing to determine that they did not spontaneously separate within the mixing tube before the separation test.

The samples were then rotated in a spin test machine for a time span of 4 minutes.

According to the results, it is possible to separate MeOH from HVO and DO, and vice versa. The results thus indicate that a disc-stack centrifugal separator is able to separate mixtures of MeOH with DO or a biodiesel, even though some components of these mixtures may be dissolvable in the MeOH. Furthermore, the results suggest that MeOH and DO or biodiesel may spontaneously separate and create layers in a storage tank if no action is undertaken.

The invention is not limited to the embodiments disclosed but may be varied and modified within the scope of the claims set out below. The invention is not limited to the type of separator as shown in the Figures. The term "centrifugal separator" also comprises centrifugal separators with a substantially horizontally oriented axis of rotation.

## Claims

1. A method (100) for cleaning fuels onboard a ship, comprising the steps of
a) supplying methanol fuel to a disc-stack centrifugal separator (2) and separating (101) a fraction of first fuel residues from said methanol fuel with said disc-stack centrifugal separator (2) and discharging a cleaned methanol phase and first fuel residues as a separate phase from said disc-stack centrifugal separator (2), thereby providing a cleaned methanol fuel onboard a ship; and
b) supplying a first fuel to the disc-stack centrifugal separator (2) and separating (105) a fraction of methanol fuel residues from said first fuel with said disc-stack centrifugal separator (2) and discharging a cleaned first fuel phase and methanol fuel residues as a separate phase from said disc-stack centrifugal separator (2), thereby providing a cleaned first fuel onboard said ship,
wherein if said first fuel has a density that is higher than the density of the methanol fuel, the disc-stack centrifugal separator (2) is operated as a purifier in step a) and as a concentrator in step b), and
wherein if said first fuel has a density that is lower than the density of the methanol fuel, the disc-stack centrifugal separator (2) is operated as a concentrator in step a) and as a purifier in step b).

2. A method (100) according to claim 1, wherein said first fuel is a diesel oil or a biofuel, or any blend thereof.

3. A method (100) according to claim 2, wherein said biofuel is selected from Fatty Acid Methyl Esters (FAME) blends and Hydrogenated Vegetable Oil (HVO).

4. A method (100) according to any previous claim, wherein the separation in step a) and step b) are performed with the same disc-stack centrifugal separator (2).

5. A method (100) according to claim 4, further comprising changing the disc-stack (19) in the disc-stack centrifugal separator (2) for switching operation of the disc-stack centrifugal separator (2) as purifier to operation of the disc-stack centrifugal separator (2) as a concentrator, and vice versa, between steps a) and b).

6. A method (100) according to claim 4, further comprising changing operational parameters for switching operation of the disc-stack centrifugal separator (2) as a purifier to operation of the disc-stack centrifugal separator (2) as a concentrator, and vice versa.

7. A method (100) according to any one of claims 1-3, wherein the separation in step a) and step b) are performed with different disc-stack centrifugal separators (2).

8. A method (100) according to any previous claim, wherein at least one of steps a) and b) is performed at a temperature of 11 °C or lower.

9. A method (100) according to any previous claim, wherein the methanol fuel that is cleaned in step a) is supplied from a storage tank (20) in which previously said first fuel has been stored, and wherein the first fuel that is cleaned in step b) is supplied from a storage tank in which previously said methanol fuel has been stored.

10. A method (100) according to claim 9, wherein the methanol fuel that is cleaned in step a) and the first fuel that is cleaned in step b) is supplied to the disc-stack centrifugal separator (2) from the same storage tank (20).

11. A method (100) according to claim 9 or 10, wherein the first fuel has a density that is higher than the density of the methanol fuel, and wherein fuel to be cleaned is initially withdrawn from a bottom portion of the storage tank (20) in step a) and initially skimmed from a top portion of the storage tank (20) in step b).

12. A method (100) according to claim 9 or 10, wherein the first fuel has a density that is lower than the density of the methanol fuel, and wherein fuel to be cleaned is initially skimmed from a top portion of the storage tank (20) in step a) and initially withdrawn from a bottom portion of the storage tank (20) in step b).

13. A method (100) according to any one of claims 10-12, further comprising emptying the storage tank (20) from methanol fuel and refilling said storage tank with the first fuel between steps a) and b).

14. A method (100) according to any previous claim, further comprising repeating steps a) and b) onboard said ship.

## Patentansprüche

1. Verfahren (100) zum Reinigen von Kraftstoffen an Bord eines Schiffes, umfassend die folgenden Schritte
a) Zuführen von Methanolkraftstoff zu einem Tellerstapel-Zentrifugalabscheider (2) und Abtrennen (101) einer Fraktion erster Kraftstoffrückstände von dem Methanolkraftstoff mit dem Tellerstapel-Zentrifugalabscheider (2) und Abgeben einer gereinigten Methanolphase und erster Kraftstoffrückstände als eine separate Phase aus dem Tellerstapel-Zentrifugalabscheider (2), wodurch ein gereinigter Methanolkraftstoff an Bord eines Schiffes bereitgestellt wird; und
b) Zuführen eines ersten Kraftstoffs zum Tellerstapel-Zentrifugalabscheider (2) und Abtrennen (105) einer Fraktion von Methanolkraftstoffrückständen von dem ersten Kraftstoff mit dem Tellerstapel-Zentrifugalabscheider (2) und Abgeben einer gereinigten ersten Kraftstoffphase und von Methanolkraftstoffrückständen als eine separate Phase aus dem Tellerstapel-Zentrifugalabscheider (2), wodurch ein gereinigter erster Kraftstoff an Bord des Schiffes bereitgestellt wird,
wobei, falls der erste Kraftstoff eine Dichte aufweist, die höher ist als die Dichte des Methanolkraftstoffs, der Tellerstapel-Zentrifugalabscheider (2) in Schritt a) als ein Reiniger und in Schritt b) als ein Konzentrator betrieben wird, und
wobei, falls der erste Kraftstoff eine Dichte aufweist, die geringer ist als die Dichte des Methanolkraftstoffs, der Tellerstapel-Zentrifugalabscheider (2) in Schritt a) als ein Konzentrator und in Schritt b) als ein Reiniger betrieben wird.

2. Verfahren (100) nach Anspruch 1, wobei der erste Kraftstoff ein Dieselöl oder ein Biokraftstoff oder ein beliebiges Gemisch davon ist.

3. Verfahren (100) nach Anspruch 2, wobei der Biokraftstoff aus Fettsäuremethylester- (FAME) Gemischen und hydriertem Pflanzenöl (HVO) ausgewählt ist.

4. Verfahren (100) nach einem vorstehenden Anspruch, wobei die Trennung in Schritt a) und Schritt b) mit demselben Tellerstapel-Zentrifugalabscheider (2) durchgeführt wird.

5. Verfahren (100) nach Anspruch 4, weiter umfassend Austauschen des Tellerstapels (19) im Tellerstapel-Zentrifugalabscheider (2) zum Umschalten des Betriebs des Tellerstapel-Zentrifugalabscheiders (2) als Reiniger zum Betrieb des Tellerstapel-Zentrifugalabscheiders (2) als ein Konzentrator und umgekehrt, zwischen den Schritten a) und b).

6. Verfahren (100) nach Anspruch 4, weiter umfassend Ändern von Betriebsparametern zum Umschalten des Betriebs des Tellerstapel-Zentrifugalabscheiders (2) als ein Reiniger zum Betrieb des Tellerstapel-Zentrifugalabscheiders (2) als ein Konzentrator und umgekehrt.

7. Verfahren (100) nach einem der Ansprüche 1-3, wobei die Trennung in Schritt a) und Schritt b) mit unterschiedlichen Tellerstapel-Zentrifugalabscheidern (2) durchgeführt wird.

8. Verfahren (100) nach einem vorstehenden Anspruch, wobei mindestens einer der Schritte a) und b) bei einer Temperatur von 11 °C oder niedriger durchgeführt wird.

9. Verfahren (100) nach einem vorstehenden Anspruch, wobei der Methanolkraftstoff, der in Schritt a) gereinigt wird, aus einem Lagertank (20) zugeführt wird, in dem zuvor der erste Kraftstoff gelagert worden ist, und wobei der erste Kraftstoff, der in Schritt b) gereinigt wird, aus einem Lagertank zugeführt wird, in dem zuvor der Methanolkraftstoff gelagert worden ist.

10. Verfahren (100) nach Anspruch 9, wobei der Methanolkraftstoff, der in Schritt a) gereinigt wird, und der erste Kraftstoff, der in Schritt b) gereinigt wird, aus demselben Lagertank (20) dem Tellerstapel-Zentrifugalabscheider (2) zugeführt werden.

11. Verfahren (100) nach Anspruch 9 oder 10, wobei der erste Kraftstoff eine Dichte aufweist, die höher ist als die Dichte des Methanolkraftstoffs, und wobei der zu reinigende Kraftstoff in Schritt a) zunächst aus einem unteren Abschnitt des Lagertanks (20) entnommen und in Schritt b) zunächst aus einem oberen Abschnitt des Lagertanks (20) abgeschöpft wird.

12. Verfahren (100) nach Anspruch 9 oder 10, wobei der erste Kraftstoff eine Dichte aufweist, die geringer ist als die Dichte des Methanolkraftstoffs, und wobei der zu reinigende Kraftstoff zunächst in Schritt a) von einem oberen Abschnitt des Lagertanks (20) abgeschöpft und in Schritt b) zunächst aus einem unteren Abschnitt des Lagertanks (20) entnommen wird.

13. Verfahren (100) nach einem der Ansprüche 10-12, weiter umfassend Entleeren des Lagertanks (20) von Methanolkraftstoff und Wiederbefüllen des Lagertanks mit dem ersten Kraftstoff zwischen den Schritten a) und b).

14. Verfahren (100) nach einem der vorstehenden Ansprüche, weiter umfassend Wiederholen der Schritte a) und b) an Bord des Schiffes.

## Revendications

1. Procédé (100) de nettoyage de combustibles à bord d'un navire, comprenant les étapes de
a) alimentation de combustible méthanol à un séparateur centrifuge à empilement de disques (2) et séparation (101) d'une fraction de premiers résidus de combustible dudit combustible méthanol avec ledit séparateur centrifuge à empilement de disques (2), et décharge d'une phase de méthanol nettoyée et de premiers résidus de combustible en tant que phase séparée à partir dudit séparateur centrifuge à empilement de disques (2), fournissant ainsi un combustible méthanol nettoyé à bord d'un navire ; et
b) alimentation d'un premier combustible au séparateur centrifuge à empilement de disques (2) et séparation (105) d'une fraction de résidus de combustible méthanol dudit premier combustible avec ledit séparateur centrifuge à empilement de disques (2), et décharge d'une première phase de combustible nettoyée et de résidus de combustible méthanol en tant que phase séparée à partir dudit séparateur centrifuge à empilement de disques (2), fournissant ainsi un premier combustible nettoyé à bord d'un navire,
dans lequel si ledit premier combustible présente une densité qui est supérieure à la densité du combustible méthanol, le séparateur centrifuge à empilement de disques (2) fonctionne en tant que purificateur à l'étape a) et en tant que concentrateur à l'étape b), et
dans lequel si ledit premier combustible présente une densité qui est inférieure à la densité du combustible méthanol, le séparateur centrifuge à empilement de disques (2) fonctionne en tant que concentrateur à l'étape a) et en tant que purificateur à l'étape b).

2. Procédé (100) selon la revendication 1, dans lequel ledit premier combustible est un gazole ou un biocombustible, ou un quelconque mélange de ceux-ci.

3. Procédé (100) selon la revendication 2, dans lequel ledit biocombustible est sélectionné parmi des mélanges d'esters méthyliques d'acides gras (FAME) et d'huile végétale hydrogénée (HVO).

4. Procédé (100) selon une quelconque revendication précédente, dans lequel la séparation à l'étape a) et à l'étape b) est réalisée avec le même séparateur centrifuge à empilement de disques (2).

5. Procédé (100) selon la revendication 4, comprenant en outre le changement de l'empilement de disques (19) dans le séparateur centrifuge à empilement de disques (2) pour passer le fonctionnement du séparateur centrifuge à empilement de disques (2) en tant que purificateur au fonctionnement du séparateur centrifuge à empilement de disques (2) en tant que concentrateur, et vice versa, entre les étapes a) et b).

6. Procédé (100) selon la revendication 4, comprenant en outre le changement de paramètres de fonctionnement pour passer le fonctionnement du séparateur centrifuge à empilement de disques (2) en tant que purificateur au fonctionnement du séparateur centrifuge à empilement de disques (2) en tant que concentrateur, et vice versa.

7. Procédé (100) selon l'une quelconque des revendications 1-3, dans lequel la séparation à l'étape a) et à l'étape b) est réalisée avec différents séparateurs centrifuges à empilement de disques (2).

8. Procédé (100) selon une quelconque revendication précédente, dans lequel au moins une parmi les étapes a) et b) est réalisée à une température de 11 °C ou moins.

9. Procédé (100) selon une quelconque revendication précédente, dans lequel le combustible méthanol qui est nettoyé à l'étape a) est fourni à partir d'un réservoir de stockage (20) dans lequel ledit premier combustible précédent a été stocké, et dans lequel le premier combustible qui est nettoyé à l'étape b) est fourni à partir d'un réservoir de stockage dans lequel ledit combustible méthanol précédent a été stocké.

10. Procédé (100) selon la revendication 9, dans lequel le combustible méthanol qui est nettoyé à l'étape a) et le premier combustible qui est nettoyé à l'étape b) est fourni au séparateur centrifuge à empilement de disques (2) à partir du même réservoir de stockage (20).

11. Procédé (100) selon la revendication 9 ou 10, dans lequel le premier combustible présente une densité qui est supérieure à la densité du combustible méthanol, et dans lequel du combustible à nettoyer est initialement retiré d'une portion inférieure du réservoir de stockage (20) à l'étape a) et initialement écumé d'une portion supérieure du réservoir de stockage (20) à l'étape b).

12. Procédé (100) selon la revendication 9 ou 10, dans lequel le premier combustible présente une densité qui est inférieure à la densité du combustible méthanol, et dans lequel du combustible à nettoyer est initialement écumé d'une portion supérieure du réservoir de stockage (20) à l'étape a) et initialement retiré d'une portion inférieure du réservoir de stockage (20) à l'étape b).

13. Procédé (100) selon l'une quelconque des revendications 10-12, comprenant en outre la vidange du réservoir de stockage (20) de combustible méthanol et le remplissage dudit réservoir de stockage avec le premier combustible entre les étapes a) et b).

14. Procédé (100) selon une quelconque revendication précédente, comprenant en outre la répétition des étapes a) et b) à bord dudit navire.
